# EUROPEAN PATENT SPECIFICATION

(11) **EP 2 175 770 B1**
(45) Date of publication and mention of the grant of the patent: **11.09.2013**
(21) Application number: 08726118.6
(22) Date of filing: 26.02.2008
(51) Int. Cl.: A61B 5/00

(54) **IMPLANTABLE VISCOSITY MONITORING DEVICE AND METHOD THEREFOR**
IMPLANTIERBARE VISKOSITÄTSÜBERWACHUNGSVORRICHTUNG UND VERFAHREN DAFÜR
DISPOSITIF DE SURVEILLANCE DE VISCOSITÉ IMPLANTABLE ET PROCÉDÉ ASSOCIÉ

(30) Priority: 23.07.2007 US 781769
(43) Date of publication of application: 21.04.2010
(73) Proprietor: Cardiac Pacemakers, Inc., St. Paul MN 55112-5798 (US)
(72) Inventor: ZHANG, Yunlong, Mounds View, MN 55112 (US); MI, Bin, Plymouth, MN 55441 (US)
(74) Representative: Pfenning, Meinig & Partner GbR
(86) International application number: PCT/US2008/002538
(87) International publication number: WO 2009/014558

(56) References cited:
- EP-A2- 0 928 598
- WO-A-92/15239
- WO-A-2004/052182
- WO-A-2005/058166
- WO-A-2006/060806
- WO-A-2007/090159
- US-A1- 2004 128 161
- US-A1- 2006 122 522
- US-A1- 2007 142 727
- DATABASE MEDLINE [Online] US NATIONAL LIBRARY OF MEDICINE (NLM), BETHESDA, MD, US March 2001 DIONISIO D P ET AL: 'Differentiation of deception using pupillary responses as an index of cognitive processing.' Database accession no. NLM11347866 & DIONISIO D P ET AL: "Differentiation of deception using pupillary responses as an index of cognitive processing.", PSYCHOPHYSIOLOGY MAR 2001, vol. 38, no. 2, March 2001 (2001-03), pages 205-211, ISSN: 0048-5772

## Description

### TECHNICAL FIELD

This patent document pertains generally to an implantable viscosity monitoring device and more particularly, but not by way of limitation, to an implantable viscosity sensor for measuring viscosity of a physiological fluid, such as blood, for instance, in contact with the implanted sensor.

### BACKGROUND

Implantable medical devices (IMDs) are devices designed to be implanted into a patient. Some examples of these devices include cardiac function management (CFM) devices such as implantable pacemakers, implantable cardioverter defibrillators (ICDs), cardiac resynchronization devices, and devices that include a combination of such capabilities. CFM devices are typically used to treat patients using electrical or other therapy. They can also help a physician or caregiver in diagnosing a patient by internal monitoring of the patient's condition. CFM devices may include one or more electrodes in communication with one or more sense amplifiers to monitor electrical heart activity within a patient. CFM devices often include one or more other physiological sensors to monitor one or more other internal patient parameters. Other examples of implantable medical devices include implantable diagnostic devices, implantable drug delivery systems, or implantable devices with neural stimulation capability.

Thrombosis is a serious clinical situation, which commonly occurs in patients with coronary artery disease (CAD), heart failure (HF), atrial fibrillation (AF), stroke, and other medical situations. Some clinical interventions, such as treatment of anemia in HF patients, increase the risk of thrombosis. Other clinical interventions, such as blood thinning intervention, increase the risk of bleeding.

Prothrombin time (PT) and International Normalized Ratio (INR) are common parameters to monitor the risk of thrombosis and bleeding. PT is the time required for a blood specimen of a patient to form a clot. Thromboplastin, a phospholipid-protein preparation that activates clotting in blood specimens, is used in determining PT. PT values can vary with use of different thromboplastins and coagulation analyzers. The INR was developed as a standardized value for indicating the risk of thrombosis and bleeding, which, unlike PT values, does not vary with the use of different thromboplastins and coagulation analyzers. For patients who need anticoagulant therapy, an acceptable INR range is between 2 and 2.5. If the INR falls below 2, it could be indicative of clotting in the patient, giving rise to concerns of a clot dislodging and leading to health complications. If the INR rises above 2.5, it could be indicative of the inability of the blood of the patient to clot. For at least these reasons, it is desirable to maintain INR between 2 and 2.5.

An implantable viscosity-sensing device based on a surface acoustic wave sensor is known from WO 2005/058166.

### OVERVIEW

The present inventors have recognized, among other things, that in at least such instances of clinical interventions such as blood thinning that increase the risk of bleeding, it is desirable to monitor the risk of thrombosis and bleeding in an ambulatory manner, in a chronic manner, or both in a chronic and ambulatory manner.

The present inventors have also recognized, among other things, that a blood viscosity measurement can be used as a surrogate parameter for PT and, if normalized, can be used as a surrogate parameter for INR. Certain examples of PT/INR measurement devices externally measure viscosity of a blood sample taken from a patient, however, such examples would not allow ambulatory or chronic blood INR measurements. Instead, such examples of external PT/INR measurement devices would require the patient to visit a clinic or laboratory, which could require relatively lengthy procedure time to obtain PT/INR value, or otherwise be inconvenient.

This document describes, among other things, an apparatus includes an implantable acoustic viscosity sensor configured to acoustically obtain a viscosity signal indicative of a viscosity of a fluid in contact with the viscosity sensor. A viscosity measurement circuit produces a viscosity measurement from the viscosity signal.

Example 1 describes an apparatus. In this example, the apparatus comprises an implantable acoustic viscosity sensor configured to acoustically obtain a viscosity signal indicative of a viscosity of a fluid in contact with the viscosity sensor. A viscosity measurement circuit is in communication with the acoustic viscosity sensor, the viscosity measurement circuit producing a viscosity measurement from the viscosity signal.

In Example 2, the apparatus of Example 1 is optionally configured such that the acoustic viscosity sensor is configured to wirelessly communicate with the viscosity measurement circuit.

In Example 3, the apparatus of one or more of Examples 1-2 optionally comprises a lead connecting the acoustic viscosity sensor with the viscosity measurement circuit, wherein the acoustic viscosity sensor is configured to communicate with the viscosity measurement circuit via the lead.

In Example 4, the apparatus of one or more of Examples 1-3 is optionally configured such that the apparatus comprises an implantable cardiac function management (CFM) device.

In Example 5, the apparatus of one or more of Examples 1-4 optionally comprises a housing including an anchor configured to anchor the housing within a subject, the acoustic viscosity sensor being carried by or coupled with the housing.

In Example 6, the apparatus of one or more of Examples 1-5 optionally is configured such that the housing can be anchored within a blood vessel of the subj ect.

In Example 7, the apparatus of one or more of Examples 1-6 is optionally configured such that the housing can be anchored within a vein of the subject.

In Example 8, the apparatus of one or more of Examples 1-7 optionally comprises an automatic drug dispenser, in communication with the viscosity measurement circuit, the drug dispenser configured to titrate delivery of a drug to a subject using the viscosity measurement as a control input.

In Example 9, the apparatus of one or more of Examples 1-8 is optionally configured such that the viscosity measurement circuit is configured to produce an International Normalized Ratio (INR) value from the viscosity measurement.

In Example 10, the apparatus of one or more of Examples 1-9 is optionally configured such that the acoustic viscosity sensor comprises a surface acoustic wave (SAW) sensor.

In Example 11, the apparatus of one or more of Examples 1-10 is optionally configured such that the acoustic viscosity sensor comprises a microelectromechanical system (MEMS) based sensor.

In Example 12, the apparatus of one or more of Examples 1-11 optionally comprises a local or remote external interface configured to be communicatively coupled to the viscosity measurement circuit or the viscosity sensor to receive information obtained from the viscosity signal.

In Example 13, the apparatus of one or more of Examples 1-12 optionally comprises the external interface being configured to display the information obtained from the viscosity signal.

In Example 14, the apparatus of one or more of Examples 1-13 optionally comprises the external interface being configured to display an International Normalized Ratio (INR) obtained using the information from the viscosity signal.

In Example 15, the apparatus of one or more of Examples 1-14 optionally comprises a separate implantable device, in addition to the acoustic viscosity sensor, the separate implantable device configured to be communicatively coupled to the viscosity measurement circuit or the viscosity sensor to receive information obtained from the viscosity signal.

In Example 16, the apparatus of one or more of Examples 1-15 optionally comprises an implantable temperature sensor configured to obtain a temperature signal, and a temperature measurement circuit in communication with the temperature sensor, the temperature measurement circuit producing a temperature measurement from the temperature signal.

Example 17 describes a method. In this example, the method comprises implantably acoustically generating a viscosity signal indicative of a viscosity of a physiological fluid of a subject, measuring a viscosity of the physiological fluid using information from the viscosity signal, and providing information about the measured viscosity to a user or process.

In Example 18, the method of Example 17 optionally comprises at least partially wirelessly communicating the viscosity signal to a viscosity measurement circuit.

In Example 19, the method of one or more of Examples 17-18 optionally comprises communicating the viscosity signal to a viscosity measurement circuit via using an at least partially intravascular lead.

In Example 20, the method of one or more of Examples 17-19 is optionally performed such that implantably acoustically generating a viscosity signal comprises using an acoustic viscosity sensor that is anchored within a subject.

In Example 21, the method of one or more of Examples 17-20 optionally comprises anchoring the acoustic viscosity sensor within a blood vessel of the subject.

In Example 22, the method of one or more of Examples 17-21 optionally is performed such that anchoring includes anchoring within a vein of the subject.

In Example 23, the method of one or more of Examples 17-22 optionally comprises automatically titrating drug delivery using the measured viscosity to control the titrating.

In Example 24, the method of one or more of Examples 17-23 optionally comprises producing an International Normalized Ratio (INR) value from the viscosity measurement.

In Example 25, the method of one or more of Examples 17-24 optionally comprises communicating, internally within the subject, information obtained from the viscosity signal.

In Example 26, the method of one or more of Examples 17-25 optionally comprises communicating, to a location external to the subject, information obtained from the viscosity signal.

In Example 27, the method of one or more of Examples 17-26 optionally comprises displaying the information obtained from the viscosity signal.

In Example 28, the method of one or more of Examples 17-27 optionally comprises displaying an International Normalized Ratio (INR) obtained from the viscosity signal.

In Example 29, the method of one or more of Examples 17-28 optionally comprises sensing a viscosity of a physiological fluid using a surface acoustic wave.

Example 30 describes an implantable medical device. In this example, the implantable medical device comprises means for implantably acoustically generating a signal indicative of a viscosity of a physiological fluid of a subject, means for measuring a viscosity of the physiological fluid using information from the viscosity signal, and means for providing information about the measured viscosity to a user or a process.

In Example 31, the device of Example 30 optionally comprises means for automatically titrating drug delivery using the viscosity measurement to control the titrating.

In Example 32, the device of one or more of Examples 30-31 optionally comprises means for communicating information obtained from the signal to a local or remote external location.

In Example 33, the device of one or more of Examples 30-32 optionally comprises means for communicating information obtained from the signal to a remote external location.

This overview is intended to provide an overview of subject matter of the present patent application. It is not intended to provide an exclusive or exhaustive explanation of the invention. The detailed description is included to provide further information about the present patent application.

A device according to the invention is defined in claim 1.

### BRIEF DESCRIPTION OF THE DRAWINGS

In the drawings, which are not necessarily drawn to scale, like numerals describe substantially similar components throughout the several views. Like numerals having different letter suffixes represent different instances of substantially similar components. The drawings illustrate generally, by way of example, but not by way of limitation, various embodiments discussed in the present document.
Fig. 1 illustrates portions of an example of a system including a leaded IMD having a viscosity sensor;
Fig. 2 illustrates an example of a leaded IMD having a viscosity sensor;
Fig. 3 illustrates portions of an example of a system including a leadless IMD having a viscosity sensor;
Figs. 4 and 5 illustrate affixation of an example of a leadless IMD having a viscosity sensor;
Figs. 6-8 illustrate examples of a leadless IMD having a viscosity sensor;
Fig. 9A illustrates a top view of an example of an acoustic wave sensor;
Fig. 9B illustrates a cross-sectional view of another example of an acoustic wave sensor;
Fig. 10 illustrates an example of a system to monitor blood viscosity of a subject and deliver a drug to the subject; and
Fig. 11 illustrates an example of a method for measuring viscosity of a physiological fluid.

### DETAILED DESCRIPTION

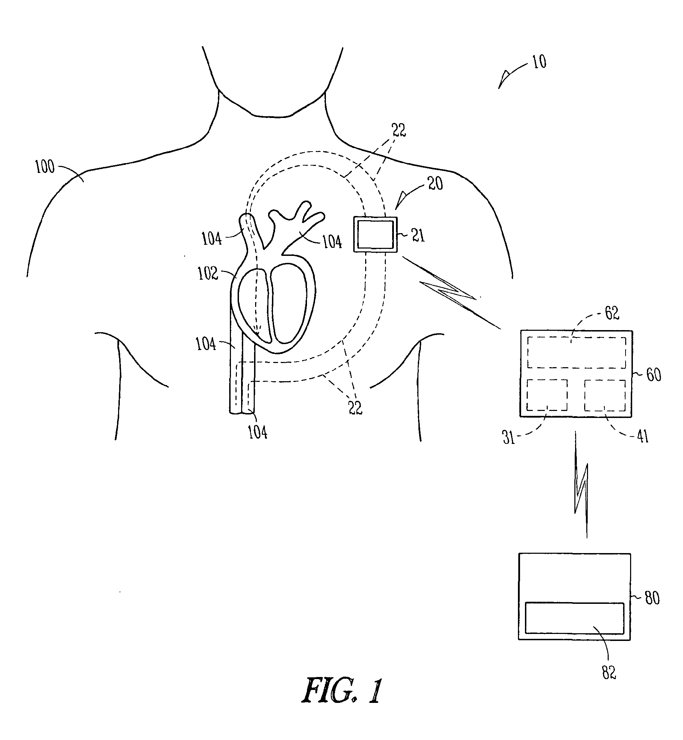

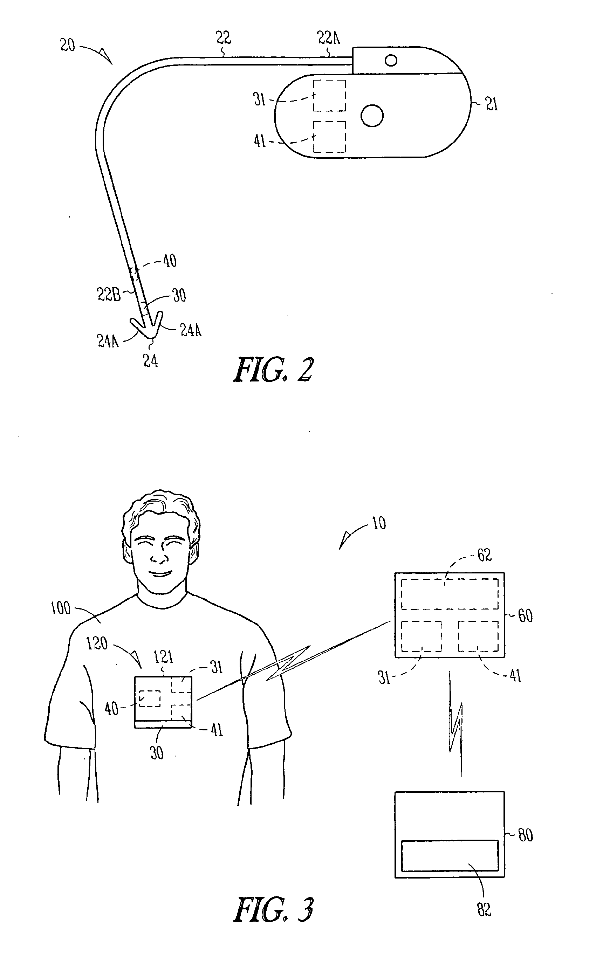

Referring to Figs. 1-3, examples of an apparatus 10 include an implantable acoustic viscosity sensor 30 configured to acoustically obtain a viscosity signal indicative of a viscosity of a fluid in contact with the viscosity sensor 30, as will be described in greater detail below. At least the acoustic viscosity sensor 30 of the apparatus 10 is configured to be implanted within a subject 100. The apparatus 10 includes, in one example, an intravascular lead 22 carrying the viscosity sensor 30 (see Figs. 1 and 2) and, in another example, includes a leadless apparatus 10 (see Fig. 3). The various configurations of the apparatus 10 are described in more detail below. The apparatus 10 includes a viscosity measurement circuit 31 in communication with the acoustic viscosity sensor 30. The viscosity measurement circuit 31 produces a viscosity measurement from the viscosity signal. In one example, the viscosity measurement circuit 31 is configured to produce an electronic representation of an International Normalized Ratio (INR) from the viscosity measurement. In one example, the viscosity measurement circuit 31 is located internally with respect to the subject 100. In another example, the viscosity measurement circuit 31 is located externally with respect to the subject 100.

Referring now to Figs. 1 and 2, an example of the leaded apparatus 10 is generally depicted, the apparatus 10 including an implantable medical device (IMD) 20 that includes an intravascular lead 22. In one example, the IMD 20 is communicatively coupled to a local external interface 60. In certain examples, the local external interface 60 can be communicatively coupled to a remote external interface 80. The IMD 20 and the local external interface 60, as well as the local external interface 60 and the remote external interface 80, can be communicatively coupled in various ways, such as over a wired or wireless telecommunications or computer network, for instance. In certain examples, the IMD 20 includes an implantable cardiac function management (CFM) device 20, such as a pacer, cardioverter, defibrillation device, cardiac resynchronization therapy (CRT) device, or combination device that combines these or other functions, such as patient monitoring, therapy control, or the like. In one example, the local external interface 60 or the remote external interface 80 is an optional element as the IMD 20 may contain all necessary hardware, circuitry, or software to perform the desired detection, processing, or therapy function(s). In another example, the local external interface 60 alone, or in combination with at least one of the remote external interface 80 and the IMD 20, performs the desired detection, processing, or therapy function(s).

In one example, the IMD 20 includes a hermetically-sealed canister or can 21. The can 21 includes, for instance, circuitry therein for coordinating operation of the IMD 20, transmitting or receiving signals, a power supply for the IMD 20, and the like. In one example, a lead 22 has a proximal end 22A coupled to the can 21, the lead 22 extending to a distal end 22B disposed at a target location within a subject 100. In one example, the acoustic viscosity sensor 30 is carried by or coupled with the lead 22. In a further example, the acoustic viscosity sensor 30 is disposed at or near the distal end 22B of the lead 22. The lead 22 can include an anchor 24 configured to anchor the lead 22 within the subject 100. In one example, the anchor 24 is disposed at or near the distal end 22B of the lead 22 and includes flexible tines 24A which are configured to hook, grab, or otherwise help attach to anatomical structure within the subject 100. The lead 22 can include other anchor configurations, such as, but not limited to, a coil; a hook; an expandable mesh, screen, or other element; or the like.

In one example, at least the distal end 22B of the lead 22 is disposed within the vasculature of the subject 100, such as with the anchor 24 configured to anchor or otherwise attach the lead 22 thereto. The lead 22 can be introduced or anchored in various areas of the vasculature of the subject 100 (at least some of which are shown in phantom in Fig. 1). In one example, the lead 22 is anchored in a blood vessel 104 of the subject 100. Depending upon the circumstances, it is contemplated that the lead 22 can be anchored in a vein or an artery. For instance, the lead 22 can be anchored in a vein, such as the superior vena cava, the inferior vena cava, or the pulmonary vein, or an artery, such as the aorta or the pulmonary artery. In another example, the lead 22 is anchored in a heart 102 of the subject 100.

The viscosity measurement circuit 31 can be disposed within the IMD 20, for instance within the can 21. In this example, the lead 22 electrically or optically connects the acoustic viscosity sensor 30 with the viscosity measurement circuit 31, wherein the acoustic viscosity sensor 30 is configured to communicate with the viscosity measurement circuit 31 via the lead 22. In another example, the viscosity measurement circuit 31 is disposed within the local external interface 60 and the acoustic viscosity sensor 30 is configured to be in wireless communication with the viscosity measurement circuit 31.

Referring to Figs. 3-8, in another example, the leadless apparatus 10 includes a leadless IMD 120. The IMD 120 of this example includes an implantable diagnostic device 120 for measuring at least one physiological parameter, such as blood viscosity, for instance. In at least one example, the IMD 120 is communicatively coupled to a local external interface 60, which, in turn is communicatively coupled to a remote external interface 80. The IMD 120 and the local external interface 60, as well as the local external interface 60 and the remote external interface 80, can be communicatively coupled in various ways, such as over a wired or wireless telecommunications or computer network, for instance. In one example, the local external interface 60 or the remote external interface 80 is an optional element as the IMD 120 may contain all necessary hardware, circuitry, or software to perform the desired detection, processing, or therapy function(s). In an example, the local external interface 60 alone or in combination with at least one of the remote external interface 80 and the IMD 120 performs the desired detection, processing, or therapy function(s). According to the invention, the IMD 120 communicates with another implantable device implanted within the subject 100, a CFM device, through an intra-body communication technique using ultrasound. In this example, the IMD 120 can relay information obtained from the viscosity signal, as generated by the IMD 120, to the other implantable device to assist the other implantable device with performance of detection, processing, or therapy functions, for instance. As discussed in more detail below, one function of the other device can include drug delivery. According to the invention, the other device is an intermediate communication station. In certain examples, it is a device to process at least the viscosity signal from the IMD 120 in order to integrate it to, for instance, a therapy function.

The IMD 120 includes a housing 121 including an anchor 124 configured to anchor the housing 121 within a subject 100. The acoustic viscosity sensor 30 is coupled with the housing 121. The housing 121 can be configured to be anchored within a blood vessel 104, such as a vein or an artery, of the subject 100. Various examples of anchoring configurations are contemplated.

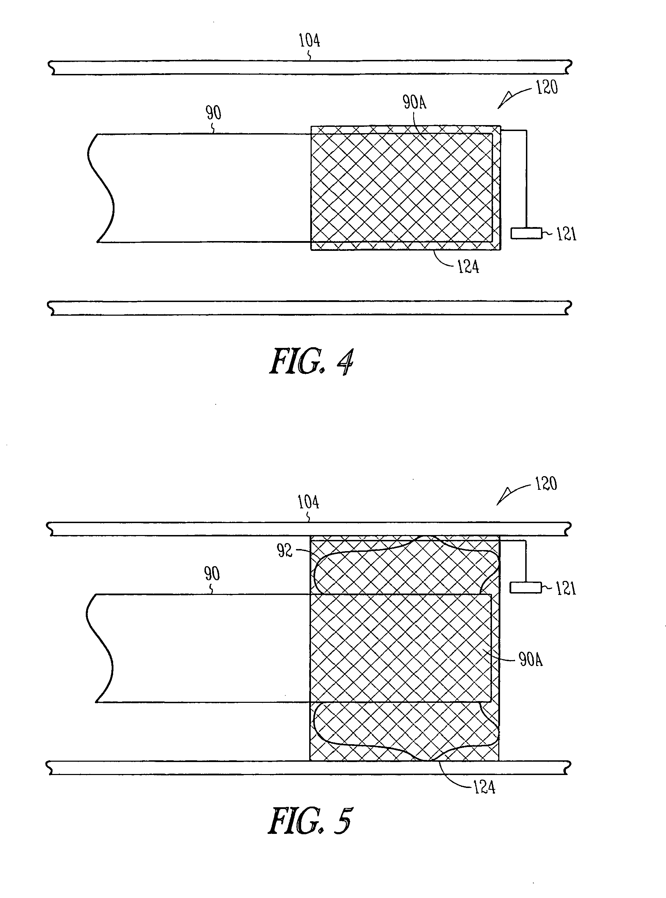

According to the invention, referring to Figs. 4 and 5, the IMD 120 includes an expandable anchor 124 having the housing 121 attached thereto. In this example, the expandable anchor 124 of the IMD 120 is coupled at or near a distal end portion 90A of a catheter 90 or other IMD delivery system. As shown, the expandable anchor 124 comprises a stent-like structure including a mesh surface that may be intravascularly delivered in a collapsed state and expanded when implanted in a blood vessel 104. To expand the expandable anchor 124, the catheter 90 may include an inflatable balloon 92, which may be inflated once the IMD 120 is positioned as desired. Inflating the balloon 92 expands the expandable anchor 124 until the expandable anchor 124 abuts a wall of the blood vessel 104. The abutting of the expandable anchor 124 with the wall of the blood vessel 104 passively fixates the expandable anchor 124 and, in turn, the IMD 120, within the blood vessel 104. Once the expandable anchor 124 is fixated within the blood vessel 104, in one example, the balloon 92 can be deflated and the catheter 90 removed from the blood vessel 104, leaving the IMD 120 in place within the blood vessel 104. In a further example, the expandable anchor 124 can be self-expanding. In this example, the balloon of the previous example need not be used to expand the expandable anchor 124. Instead, for instance, the expandable anchor 124 can be retained in a compressed state on the catheter 90. The expandable anchor 124 can be released from the catheter 90 at a desired location, at which point the expandable anchor 124 can self-expand from its compressed state to abut the wall of the blood vessel 104 to fixate the expandable anchor 124 and, in turn, the IMD 120, within the blood vessel 104.

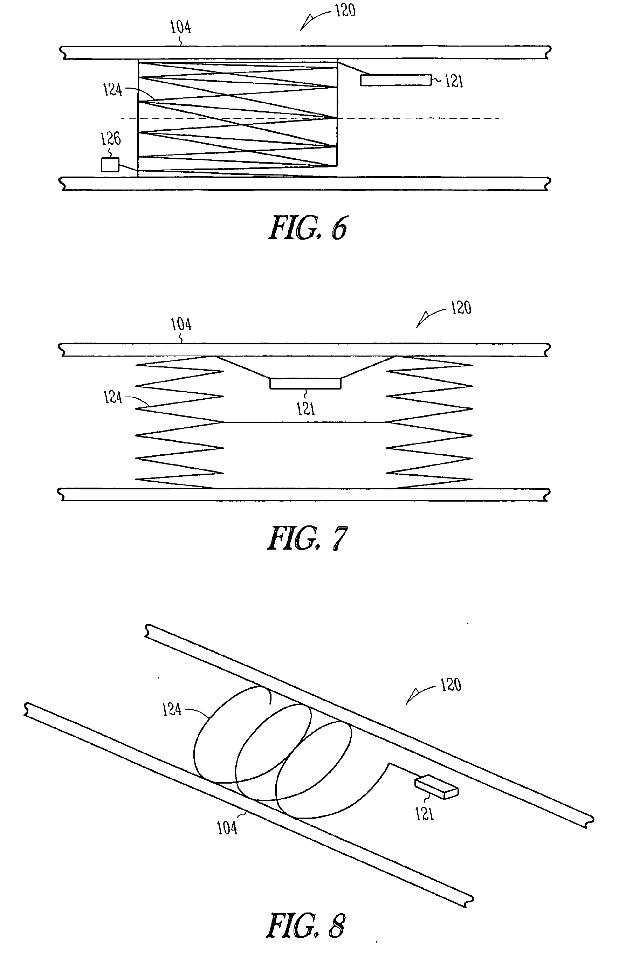

Referring to Fig. 6, the IMD 120 includes another example of an expandable anchor 124. In this example, the expandable anchor 124 includes a zigzag-like configuration that is in contact with an inner surface of the blood vessel 104. Additionally, the example shown in Fig. 6 includes a second attached element 126 That is, the IMD 120 includes an element in addition to housing 121 including the acoustic viscosity sensor 30. In one example, the second element 126 includes a power source, such as a battery, for instance, for powering at least the IMD 120 and the acoustic viscosity sensor 30 thereof. In another example, the second element 126 includes a temperature sensor for monitoring a blood temperature, as will be described in more detail below. In other examples, the second element 126 includes another monitoring device, such as a flow sensor for monitoring blood flow. In yet another example, the second element 126 includes a combination of devices therein. In still another example, one or more of the devices described above with respect to the second element 126 can be included within a single element, such as the housing 121. The connection between one or more of the expandable anchor 124, the housing 121, or the second element 126 may be achieved mechanically such as using one or more crimps, adhesives, welding, or any other convenient mechanism or material.

Referring to Fig. 7, the IMD 120 includes yet another example not according to the invention of an expandable anchor 124. The expandable anchor 124 of this example includes two expandable portions with the housing 121 disposed therebetween.

Referring to Fig. 8, the IMD 120 includes still another example not according to the invention of an expandable anchor 124. The expandable anchor 124 of this example includes a coil-like configuration. Other expandable electrode configurations can be used.

The insertion of the IMD 120 of the examples shown in Figs. 6-8 into the blood vessel 104 may be performed in a variety of ways. In one example, the insertion of the IMD 120 is performed via a catheterization procedure, such as the delivery system described above and shown in Figs. 4 and 5. In such an example, the IMD 120 may be mounted on a delivery system in a compressed configuration so as to enable navigation to the desired blood vessel 104. At the desired deployment site, the expandable anchor 124 may then be allowed to expand to abut a wall of the blood vessel 104. In another example, the IMD 120 is inserted into an incision in the blood vessel 104.

As seen in the examples shown in Figs. 1-3, each of the lead-including IMD 20 and the leadless IMD 120 includes an acoustic viscosity sensor 30 for sensing viscosity of a physiological fluid, such as blood, for instance. Several types of acoustic viscosity sensors 30 are contemplated herein.

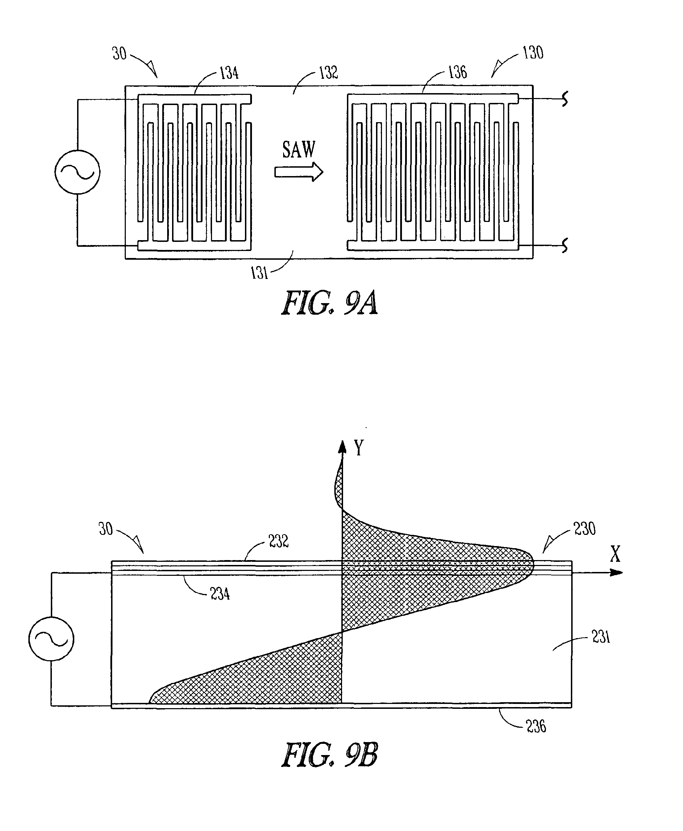

Referring to Fig. 9A, in one example, the acoustic viscosity sensor 30 comprises a piezoelectric surface acoustic wave (SAW) sensor 130. In this example, a surface 132 includes a piezoelectric layer 131 having coupled thereto input interdigitated electrodes 134, output interdigitated electrodes 136, and an insulation layer. In one example, at least the input and output electrodes 134, 136 are coupled to a top of the piezoelectric layer 131. In this example, at least one of interdigitated electrodes 134 is driven with, for instance, an alternating voltage signal to activate the SAW transducer and generate surface acoustic wave along the surface 132 at a frequency. The vibrating surface 132 is in contact with a fluid, such as blood. In this example, the viscosity of the fluid alters the oscillation frequency of the surface 132. For instance, an increased fluid viscosity results in a lower oscillation frequency, and a decreased fluid viscosity results in a higher oscillation frequency. The fluid in contact with the surface 132 also causes resonance damping and an insertion loss due to the acoustic wave transferred to the fluid, which can be related to the viscosity of the fluid. This oscillation frequency shift or the power insertion loss can be used to create a viscosity signal, which can be converted into a viscosity measurement of the fluid. In certain examples, SAW sensors 130 have different surface acoustic wave modes by choosing different piezoelectric material orientations. For instance, in one example, the sensor 130 operates in a shear vertical surface acoustic wave (SV-SAW) mode in which transverse displacement of the SAW is normal to the surface 132. In one example, the sensor 130 operates in a shear horizontal surface acoustic wave (SH-SAW) mode in which transverse displacement of the SAW is parallel to the surface 132.

Referring to Fig. 9B, in another example not according to the invention, the acoustic viscosity sensor 30 comprises a bulk acoustic wave (BAW) sensor 230. Examples of BAW sensors include, for instance, a thickness shear mode (TSM) resonator and a shear-horizontal acoustic plate mode (SH-APM) sensor. In certain examples, the BAW sensor 230 includes a piezoelectric layer 231 sandwiched between top and bottom thin film electrodes 234, 236. In this example, an alternating voltage is applied to the electrodes 234, 236 to vibrate the piezoelectric layer 231 in a thickness shear mode at a frequency. Fluid, such as blood, in contact with the vibrating surface 232 mechanically interacts with the vibrating surface 232. A curve is depicted in Fig. 9B that represents displacements across a cross section of the BAW sensor 230, the fluid, and a solid-liquid interface therebetween. It is contemplated that the surface 232 is vibrated at the fundamental frequency, although it should be understood that other frequencies can be used or can result, such as harmonics. As in the example of the piezoelectric SAW sensor 130 above, the viscosity of the fluid alters the oscillation frequency of acoustic wave, with, for instance, an increased fluid viscosity resulting in a lower oscillation frequency and a decreased fluid viscosity resulting in a higher oscillation frequency. The fluid in contact with the surface 232 causes resonance damping and a frequency shift, which can be related to the viscosity of the fluid. In an example, using the frequency change, the acoustic viscosity sensor 230 creates a signal that can be converted into a viscosity measurement of the fluid.

In one example, the acoustic viscosity sensor 30 comprises a microelectromechanical system (MEMS) based sensor. In one example, the MEMS based sensor comprises a solid-state acoustic wave transducer that is manufactured using a micro-machining process. In one example, the MEMS sensor comprises a solid-state surface acoustic wave (SAW) transducer. In another example, the MEMS sensor comprises a solid-state bulk acoustic wave (BAW) transducer. In these examples, a transducer of either of the acoustic sensors 130, 230 can be manufactured together with signal processing or conditioning electronics in one die. In another example, a transducer of either of the sensors 130, 230 can be packaged together with signal processing or conditioning electronics in one package. In another example, the acoustic viscosity sensor 30 includes only the transducer of either of the acoustic sensors 130, 230, with the signal processing or conditioning electronics located in another device, either within or outside of the subject 100. In one example, the sensor and electronics are packaged in a titanium or other biocompatible material housing or box with the sensing surface exposed. In certain examples, the sensor packaging includes a coating of a drug eluting substance. In one example, the sensor packaging includes a coating of a drug eluting substance at least at the sensing surface.

Referring again to Figs. 1-3, in other examples, the apparatus 10 includes an implantable temperature sensor 40 configured to obtain a temperature signal. In one example, the leaded IMD 20 includes the temperature sensor 40 disposed in or on the lead 22. In one example, the temperature sensor 40 is disposed at or near the distal end 22B of the lead 22. In another example, the leadless IMD 120 includes the temperature sensor 40 within the housing 121. In one example, the temperature sensor 40 is configured to sense blood temperature. A temperature measurement circuit 41 is in communication with the temperature sensor 40. As with the viscosity measurement circuit 31 described above, the temperature measurement circuit 41 can be disposed within the IMD 20, 120 or within the local external interface 60. In one example, the temperature measurement circuit 41 of the lead including IMD 20 is disposed within the can 21 and is in communication with the temperature sensor 40 via the lead 22. In another example, the temperature measurement circuit 41 of the leadless IMD 120 is disposed within the housing 121 so as to be in direct communication with the temperature sensor 40, which is also disposed within the housing 121. In yet another example, the temperature measurement circuit 41 of the apparatus 10 is disposed within the local external interface 60 and is in wireless communication with the temperature sensor 40 of the IMD 20, 120. The temperature measurement circuit 41 is configured to produce a temperature measurement from the temperature signal. The temperature measurement obtained from the temperature sensor 40 can then be used in monitoring or therapy of the subject 100. For instance, the temperature measurement can be used in the blood viscosity assessment to take into account temperature-related changes in the viscosity measurement. In an example, the temperature measurement is used in other aspects of monitoring or therapy and is included with the IMD 20, 120 to avoid having to implant a separate temperature-sensing END. In other examples, the temperature measurement circuit 41 is included on the same circuit board as the viscosity measurement circuit 31 to limit manufacturing costs associated therewith and to maximize use of space within the IMD 20, 120.

Referring to Figs. 1 and 3, the apparatus 10 can optionally include the local external interface 60 and the remote external interface 80. The local external interface 60 can comprise a handheld reader, a personal digital assistant (PDA), or a desktop or laptop computer. Information derived from the viscosity signal obtained from the IMD 20, 120 can be communicated to the local external interface 60 or the remote external interface 80 to perform viscosity processing at such other locations. Moreover, such processing can include information from one or more devices, either implanted within or externally situated with respect to the subject 100. For example, a blood viscosity measurement as measured by the IMD 20, 120 can be combined with information obtained from an implantable cardiac function management device, for instance, during processing at the remote external interface 80, such as to trigger an alert or responsive therapy.

Additionally, in at least one example, at least one of the local external interface 60 and the remote external interface 80 is configured to allow a user (for instance, the subject 100, a physician, or a caregiver) to program the IMD 20, 120. For instance, the user can program operation modes of the IMD 20, 120, such as monitoring time of the viscosity sensor 30 and the optional temperature sensor 40. Monitoring can include, but is not limited to, continuous monitoring, recurrent or periodic monitoring, or sleep/wake-up monitoring, which monitors changes in blood viscosity and, optionally, temperature during the transitional periods between sleep and wakefulness.

In certain examples, as stated above, information from the IMD 20, 120 can be communicated to the local external interface 60 or the remote external interface 80. The local external interface 60 or the remote external interface 80 can be configured to store or display the information.

In one example, the local external interface 60 is configured to receive information obtained from the viscosity signal. The local external interface 60 can be configured to display the information obtained from the viscosity signal. For instance, the local external interface 60 optionally includes a display 62 to display the information. The display 62 can take various forms, including, but not limited to, a monitor, a liquid crystal display (LCD), or a light emitting diode (LED) display. The information can be portrayed in other forms, including a printout from a printer, an audible alert or signal such as a beep or buzz, or an alert light such as a blinking light. In one example, the local external interface 60 is configured to display the International Normalized Ratio (INR) obtained using the information from the viscosity signal. In other examples, information other than the INR is displayed by the display 62 of the local external interface 60, such as the viscosity measurement, prothrombin time (PT), or blood temperature as measured by the temperature sensor 40. Additionally, the display 62 can portray other information, such as information obtained from other IMDs or other components of the IMD 20, 120; externally measured information, such as a weight measurement; or programmed settings of the IMD 20, 120.

In another example, the remote external interface 80 is configured to be communicatively coupled to receive the information obtained from the viscosity signal. The remote external interface 80 can comprise, for instance, a remote server or computer. The remote external interface 80 is configured to display the information obtained from the viscosity signal. For instance, the remote external interface 80 optionally includes a display 82 to display the information. The display 82 can take various forms, including, but not limited to, a monitor, a liquid crystal display (LCD), or a light emitting diode (LED) display. The information can be portrayed in other forms, including a printout from a printer, an audible alert or signal such as a beep or buzz, or an alert light such as a blinking light. In one example, the remote external interface 80 is configured to display the International Normalized Ratio (INR) obtained using the information from the viscosity signal. In other examples, information other than the INR is displayed by the display 82 of the remote external interface 80, such as the viscosity measurement, prothrombin time (PT), or blood temperature as measured by the temperature sensor 40. Additionally, the display 82 can portray other information, such as information obtained from other IMDs or other components of the IMD 20, 120; externally measured information, such as a weight measurement; or programmed settings of the IMD 20, 120. The remote external interface 80 can be accessible to a physician or caregiver to receive the information obtained by the IMD 20, 120 and to allow the physician or caregiver to alter the settings of the IMD 20, 120 remotely, contact the subject 100 to discuss the information received, or contact other medical professionals (emergency medical technicians, for instance) to provide the subject 100 with medical treatment.

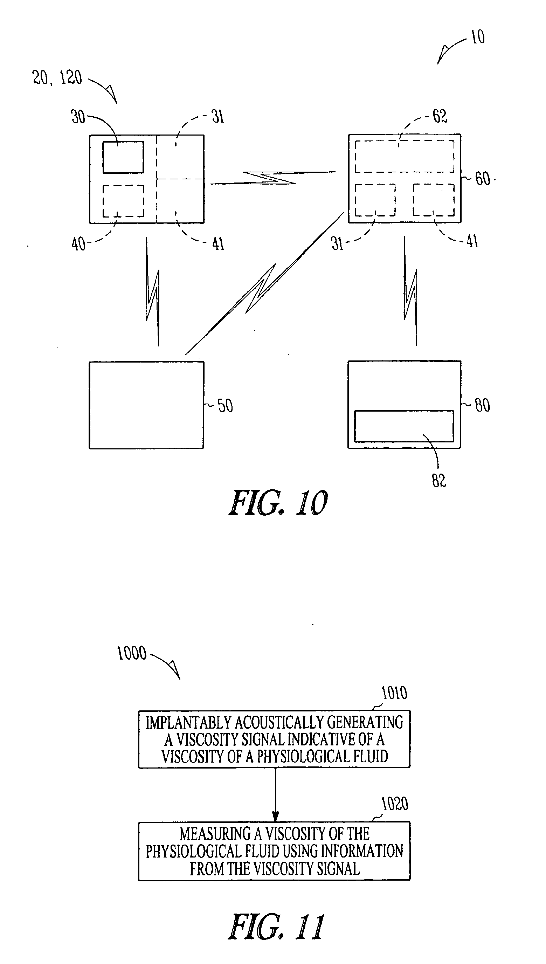

Referring to Fig. 10, in another example, the apparatus 10 includes an automatic drug dispenser 50 configured to titrate delivery of a drug to the subject 100 (Figs. 1 and 3) using the viscosity measurement as a control input. In one example, the automatic drug dispenser 50 is carried by the subject 100 and is communicatively coupled to the IMD 20, 120, the local external interface 60, or the remote external interface 80. In one example, if the viscosity measurement or INR reaches, falls below, or rises above a threshold value, the automatic drug dispenser 50 can be configured to increase or decrease the dosage of or otherwise administer a drug, such as an anti-coagulant or blood thinner drug, to the subject 100. The threshold is associated with or related to INR values or prothrombin time. For instance, if an INR reading of the subject 100 falls below 2, the IMD 20, 120, the local external interface 60, or the remote external interface 80 can communicate with the automatic drug dispenser 50 to deliver a blood thinner drug to the subject 100. In this example, the IMD 20, 120 can continue monitoring blood viscosity, and, if the INR does not rise above 2, the automatic drug dispenser 50 can be operated to deliver an additional dose of the blood thinner drug. In this way, the apparatus 10 can be used to maintain the INR of the subject 100 within an acceptable range in an effort to limit negative health complications to the subject 100, such as stroke, heart failure, and other medical situations. In one example, if the viscosity measurement or INR reaches, falls below, or rises above a threshold value, the apparatus 10 provides an alert to the subject 100 to allow the subject 100 to ingest a blood thinner drug in lieu of automatic drug titration. In one example, the threshold is a range that includes an upper bound and a lower bound to assist with or guide treatment for bleeding or thrombosis risks.

Fig. 11 shows an example of a method 1000. At 1010, a viscosity signal is implantably acoustically generated to be indicative of a viscosity of a physiological fluid, such as, for instance, blood, although the present example is not intended to be limited as such. In one example, this includes sensing a viscosity of a physiological fluid using a surface acoustic wave. For instance, the viscosity signal can be generated by the acoustic viscosity sensor 30 of the IMD 20, 120. At 1020, a viscosity of the physiological fluid is measured using information from the viscosity signal. For instance, the viscosity measurement can be generated by the viscosity measurement circuit 31. In one example, the viscosity signal is wirelessly or otherwise communicated to a viscosity measurement circuit 31. In an example, the viscosity signal is communicated to a viscosity measurement circuit 31 using an at least partially intravascular lead 22.

In further examples, the method 1000 includes anchoring an acoustic viscosity sensor 30 within a subject 100. For instance, the IMD 20, 120 including the acoustic viscosity sensor 30 can be anchored within the subject 100 using an anchor 24, 124. In one example, anchoring of the acoustic viscosity sensor 30 includes anchoring within a blood vessel 104 of the subject 100. In one example, anchoring includes anchoring within a vein 104 of the subject 100. In other examples, anchoring includes anchoring within an artery 104 or heart 102 of the subject 100.

In certain examples, the method 1000 includes producing an electronic representation of an International Normalized Ratio (INR) from the viscosity measurement. In other examples, other information is produced, such as an electronic representation of prothrombin time (PT) from the viscosity measurement or a temperature measurement from a temperature signal of the temperature sensor 40. In one example, a portion of the apparatus 10, such as the viscosity measurement circuit 31, for instance, correlates the viscosity measurement with electronic representations of INR or PT values. Such electronic representations of INR or PT values can be stored in a database of a memory element of a portion of the apparatus, such as, for instance, the viscosity measurement circuit 31. In one example, the method 1000 includes communicating information obtained from the viscosity signal to an external location. For instance, the information can be communicated to the local external interface 60 or the remote external interface 80. In a further example, the method 1000 includes displaying the information obtained from the viscosity signal. For instance, the information can be displayed on the display 62 of the local external interface 60 or the display 82 of the remote external interface 80. In one example, the method 1000 includes displaying an electronic representation of the International Normalized Ratio (INR) obtained from the viscosity signal. In another example, the INR obtained from the viscosity signal is audibly communicated using one or more beeps, buzzes, or other such sounds.

In another example, the method 1000 includes titrating drug delivery using the measured viscosity to control the titrating. For instance, drug delivery can be titrated by the automatic drug dispenser 50 using at least the viscosity measurement as a control input.

Advantageously, the apparatus 10 provides for internal measurement of blood viscosity. That is, the apparatus 10 does not require the subject 100 to visit a clinic or laboratory and have a blood sample taken in order to obtain a blood viscosity measurement. Further advantageously, the apparatus 10 provides real-time measurement of blood viscosity from which PT and INR values can be derived so that the subject 100 need not wait for a relatively lengthy procedure time to obtain a PT or INR value.

### Some Notes

The above detailed description includes references to the accompanying drawings, which form a part of the detailed description. The drawings show, by way of illustration, specific embodiments in which the invention can be practiced. These embodiments are also referred to herein as "examples." In the event of inconsistent usages between this document and those documents so incorporated by reference, the usage in the incorporated reference(s) should be considered supplementary to that of this document; for irreconcilable inconsistencies, the usage in this document controls.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In the appended claims, the terms "including" and "in which" are used as the plain-English equivalents of the respective terms "comprising" and "wherein." Also, in the following claims, the terms "including" and "comprising" are open-ended, that is, a system, device, article, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

Method examples described herein can be computer or machine-implemented at least in part. Some examples can include a computer-readable medium or machine-readable medium encoded with instructions operable to configure an electronic device to perform methods as described in the above examples. An implementation of such methods can include code, such as microcode, assembly language code, a higher-level language code, or the like. Such code can include computer readable instructions for performing various methods. The code may form portions of computer program products. Further, the code may be tangibly stored on one or more volatile or non-volatile computer-readable media during execution or at other times. These computer-readable media may include, but are not limited to, hard disks, removable magnetic disks, removable optical disks (e.g., compact disks and digital video disks), magnetic cassettes, memory cards or sticks, random access memories (RAM's), read only memories (ROM's), and the like.

The above description is intended to be illustrative, and not restrictive. For example, the above-described examples (or one or more aspects thereof) may be used in combination with each other. Other embodiments can be used, such as by one of ordinary skill in the art upon reviewing the above description. The Abstract is provided to comply with 37 C.F.R. §1.72(b), to allow the reader to quickly ascertain the nature of the technical disclosure. It is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description, with each claim standing on its own as a separate embodiment. The scope of the invention should be determined with reference to the appended claims, along with the full scope of equivalents to which such claims are entitled.

## Claims

1. An apparatus (10), comprising:
an implantable viscosity-sensing device (120) including:
a housing;
an implantable surface acoustic wave (SAW) viscosity sensor (30) coupled with the housing (121), the SAW viscosity sensor (120) being configured to acoustically obtain a viscosity signal indicative of a viscosity of a fluid in contact with the viscosity sensor (30); and
a viscosity measurement circuit (31) coupled with the housing (121), the viscosity measurement circuit (31) in communication with the acoustic viscosity sensor (30), the viscosity measurement circuit (31) producing a viscosity measurement from the viscosity signal; and
an anchor (124) coupled with the housing and configured to anchor the housing within a blood vessel (104) of a subject, the anchor (124) including a stent-like expandable mesh surface configured to be intravascularly delivered in a collapsed state and expanded to fixate the implantable viscosity-sensing device (120) within the blood vessel;
an implantable cardiac function management (CFM) device, physically separate from and in addition to the implantable viscosity-sensing device (120), the implantable CFM device configured to be ultrasonically communicatively coupled to the implantable viscosity-sensing device (120) to receive information obtained from the viscosity signal; and
an external interface (60, 80) configured to be communicatively coupled to the implantable viscosity-sensing device over a telecommunications or computer network, the implantable CFM device being an intermediate communication station, to receive the information obtained from the viscosity signal, the external interface (60, 80) configured to display an International Normalized Ratio (INR) obtained using the information from the viscosity signal.

2. The apparatus (10) of claim 1, wherein the acoustic viscosity sensor (30) is configured to wirelessly communicate with the viscosity measurement circuit (31).

3. The apparatus (10) of claim 1, comprising a lead (22) connecting the acoustic viscosity sensor (30) with the viscosity measurement circuit (31), wherein the acoustic viscosity sensor (30) is configured to communicate with the viscosity measurement circuit (3 1) via the lead.

4. The apparatus (10) of any of claims 1 through 3, comprising an automatic drug dispenser (50), in communication with the viscosity measurement circuit (31), the drug dispenser (50) configured to titrate delivery of a drug to a subject using the viscosity measurement as a control input.

5. The apparatus (10) of any of claims 1 through 4, wherein the viscosity measurement circuit (31) is configured to produce an International Normalized Ratio (INR) value from the viscosity measurement.

6. The apparatus (10) of any of claims 1 through 5, wherein the acoustic viscosity sensor (30) comprises a microelectromechanical system (MEMS) based sensor.

7. The apparatus (10) of any of claims 1 through 6, wherein the external interface (60, 80) is configured to be communicatively coupled to the viscosity measurement circuit (31) or the viscosity sensor (30) to receive information obtained from the viscosity signal.

8. The apparatus (10) of any of claims 1 through 7, wherein the external interface (60, 80) is configured to display the information obtained from the viscosity signal.

9. The apparatus (10) of any of claims 1 through 8, comprising a separate implantable device, in addition to the acoustic viscosity sensor (30), the separate implantable device (50) configured to be communicatively coupled to the viscosity measurement circuit (31) or the viscosity sensor (30) to receive information obtained from the viscosity signal.

10. The apparatus of any of claims 1 through 9, comprising:
an implantable temperature sensor (40) configured to obtain a temperature signal; and
a temperature measurement circuit (41) in communication with the temperature sensor (40), the temperature measurement circuit (41) producing a temperature measurement from the temperature signal.

## Patentansprüche

1. Vorrichtung (10), welche aufweist:
eine implantierbare, viskositätserfassende Vorrichtung (120), enthal-tend:
ein Gehäuse;
einen implantierbaren, akustischen Oberflächenwellen(SAW)-Viskositätssensor (30), der mit dem Gehäuse (121) gekoppelt ist, wobei der SAW-Viskositätssensor (120) ausgebildet ist zum akustischen Erhalten eines Viskositätssignals, das eine Viskosität eines Fluids in Kontakt mit dem Viskositätssensor (30) anzeigt; und
eine Viskositätsmessschaltung (31), die mit dem Gehäuse (121) gekoppelt ist, wobei die Viskositätsmessschaltung (31) in Kommunikation mit dem akustischen Viskositätssensor (30) ist und die Viskositätsmessschaltung (31) eine Viskositätsmessung anhand des Viskositätssignals erzeugt; und
einen Anker (124), der mit dem Gehäuse gekoppelt ist und ausgebildet ist, das Gehäuse innerhalb eines Blutgefäßes (104) eines Subjekts zu verankern, wobei der Anker (124) eine stentartige, expandierbare Maschenoberfläche hat und ausgebildet ist, in einem zusammengelegten Zustand intravaskulär zugeführt und expandiert zu werden, um die implantierbare, viskositätserfassende Vorrichtung (120) innerhalb des Blutgefäßes zu fixieren;
eine implantierbare Herzfunktionsverwaltungs(CFM)-Vorrichtung, die körperlich getrennt von und zusätzlich zu der implantierbaren Viskositätserfassungsvorrichtung (120) vorgesehen ist, wobei die implantierbare CFM-Vorrichtung ausgebildet ist, ultraschallmäßig kommunikativ mit der implantierbaren Viskositätserfassungsvorrichtung (120) gekoppelt zu sein, um aus dem Viskositätssignal erhaltene Informationen zu empfangen; und
eine externe Schnittstelle (60, 80), die ausgebildet ist, über ein Telekommunikations- oder Computernetz kommunikativ mit der implantierbaren Viskositätserfassungsvorrichtung gekoppelt zu sein, wobei die implantierbare CFM-Vorrichtung eine Zwischenkommunikationsstation ist, um die aus dem Viskositätssignal erhaltenen Informationen zu empfangen, wobei die externe Schnittstelle (60, 80) ausgebildet ist zum Anzeigen eines international normierten Verhältnisses (INR), das unter Verwendung der Informationen aus dem Viskositätssignal erhalten wurde.

2. Vorrichtung (10) nach Anspruch 1, bei der der akustische Viskositätssensor (30) ausgebildet ist zum drahtlosen Kommunizieren mit der Viskositätsmessschaltung (31).

3. Vorrichtung (10) nach Anspruch 1, aufweisend einen Leiter (22), der den akustischen Viskositätssensor (30) mit der Viskositätsmessschaltung (31) verbindet, wobei der akustische Viskositätssensor (30) ausgebildet ist, mit der Viskositätsmessschaltung (31) über den Leiter zu kommunizieren.

4. Vorrichtung (10) nach einem der Ansprüche 1 bis 3, aufweisend eine automatische Medikamentenabgabevorrichtung (50) in Verbindung mit der Viskositätsmessschaltung (31), wobei die Medikamentenabgabevorrichtung (50) ausgebildet ist, die Zuführung eines Medikaments zu einem Subjekt unter Verwendung der Viskositätsmessung als einer Steuereingabe zu titrieren.

5. Vorrichtung (10) nach einem der Ansprüche 1 bis 4, bei der die Viskositätsmessschaltung (31) ausgebildet ist zum Erzeugen eines international normierten Verhältnis(INR)-Wertes anhand der Viskositätsmessung.

6. Vorrichtung (10) nach einem der Ansprüche 1 bis 5, bei der der akustische Viskositätssensor (30) einen auf einem mikroelektromechanischen System (MEMS) basierenden Sensor aufweist.

7. Vorrichtung (10) nach einem der Ansprüche 1 bis 6, bei der die externe Schnittstelle (60, 80) ausgebildet ist, kommunikativ mit der Viskositätsmessschaltung (31) oder dem Viskositätssensor (30) gekoppelt zu sein, um aus dem Viskositätssignal erhaltene Informationen zu empfangen.

8. Vorrichtung (10) nach einem der Ansprüche 1 bis 7, bei der die externe Schnittstelle (60, 80) ausgebildet ist, die aus dem Viskositätssignal erhaltenen Informationen anzuzeigen.

9. Vorrichtung (10) nach einem der Ansprüche 1 bis 8, aufweisend eine getrennte implantierbare Vorrichtung zusätzlich zu dem akustischen Viskositätssensor (30), wobei die getrennte implantierbare Vorrichtung (50) ausgebildet ist, kommunikativ mit der Viskositätsmessschaltung (31) oder dem Viskositätssensor (30) gekoppelt zu sein, um aus dem Viskositätssignal erhaltene Informationen zu empfangen.

10. Vorrichtung nach einem der Ansprüche 1 bis 9, welche aufweist:
einen implantierbaren Temperatursensor (40), der ausgebildet ist, ein Temperatursignal zu erhalten; und
eine Temperaturmessschaltung (41) in Verbindung mit dem Temperatursensor (40), wobei die Temperaturmessschaltung (41) eine Temperaturmessung anhand des Temperatursignals erzeugt.

## Revendications

1. Appareil (10), comprenant :
un dispositif de détection de viscosité implantable (120) incluant :
un boîtier ;
un capteur de viscosité (30) par ondes acoustiques de surface (SAW) implantable couplé au boîtier (121), le capteur de viscosité SAW (120) étant configuré de manière à pouvoir obtenir acoustiquement un signal de viscosité indicatif d'une viscosité d'un fluide en contact avec le capteur de viscosité (30) ; et
un circuit de mesure de viscosité (31) couplé au boîtier (121), le circuit de mesure de viscosité (31) étant en communication avec le capteur de viscosité acoustique (30), le circuit de mesure de viscosité (31) produisant une mesure de viscosité à partir du signal de viscosité ; et
un ancrage (124) couplé au boîtier et configuré de manière pouvoir ancrer le boîtier à l'intérieur d'un vaisseau sanguin (104) d'un sujet, l'ancrage (124) incluant une surface de maillage extensible en forme de stent configurée de manière à pouvoir être délivrée intravasculairement dans un état affaissé et à pouvoir être étendue afin de fixer le dispositif de détection de viscosité implantable (120) à l'intérieur du vaisseau sanguin ;
un dispositif de gestion de fonction cardiaque (CFM) implantable, physiquement séparé du dispositif de détection de viscosité implantable (120) et en plus de celui-ci, le dispositif CFM implantable étant configuré de manière à pouvoir être couplé en communication par ultrasons au dispositif de détection de viscosité implantable (120) pour recevoir une information obtenue à partir du signal de viscosité ; et
une interface externe (60, 80) configurée de manière à pouvoir être couplée en communication au dispositif de détection de viscosité implantable sur un réseau de télécommunication ou d'ordinateurs, le dispositif CFM implantable étant une station de communication intermédiaire, pour recevoir l'information obtenue à partir du signal de viscosité, l'interface externe (60, 80) étant configurée de manière à pouvoir afficher un Rapport Normalisé International (INR) obtenu en utilisant l'information issue du signal de viscosité.

2. Appareil (10) selon la revendication 1, dans lequel le capteur de viscosité acoustique (30) est configuré de manière à pouvoir communiquer sans fil avec le circuit de mesure de viscosité (31).

3. Appareil (10) selon la revendication 1, comprenant une connexion (22) qui connecte le capteur de viscosité acoustique (30) avec le circuit de mesure de viscosité (31), dans lequel le capteur de viscosité acoustique (30) est configuré de manière à pouvoir communiquer avec le circuit de mesure de viscosité (31) via la connexion.

4. Appareil (10) selon l'une quelconque des revendications 1 à 3, comprenant un dispositif de délivrance de médicament (50), en communication avec le circuit de mesure de viscosité (31), le dispositif de délivrance de médicament (50) étant configuré de manière à pouvoir titrer la délivrance d'un médicament à un sujet en utilisant la mesure de viscosité en tant qu'entrée de commande.

5. Appareil (10) selon l'une quelconque des revendications 1 à 4, dans lequel le circuit de mesure de viscosité (31) est configuré de manière à pouvoir produire une valeur de Rapport Normalisé International (INR) à partir de la mesure de viscosité.

6. Appareil (10) selon l'une quelconque des revendications 1 à 5, dans lequel le capteur de viscosité acoustique (30) comprend un capteur basé sur un système microélectromécanique (MEMS).

7. Appareil (10) selon l'une quelconque des revendications 1 à 6, dans lequel la surface externe (60, 80) est configurée de manière à pouvoir être couplée en communication au circuit de mesure de viscosité (31) ou au capteur de viscosité (30) pour recevoir une information obtenue à partir du signal de viscosité.

8. Appareil (10) selon l'une quelconque des revendications 1 à 7, dans lequel la surface externe (60, 80) est configurée de manière à pouvoir afficher l'information obtenue à partir du signal de viscosité.

9. Appareil (10) selon l'une quelconque des revendications 1 à 8, comprenant un dispositif implantable séparé, en plus du capteur de viscosité acoustique (30), le dispositif implantable séparé (50) étant configuré de manière à pouvoir être couplé en communication au circuit de mesure de viscosité (31) ou au capteur de viscosité (30) pour recevoir une information obtenue à partir du signal de viscosité.

10. Appareil selon l'une quelconque des revendications 1 à 9, comprenant :
un capteur de température implantable (40) configuré de manière à pouvoir obtenir un signal de température ; et
un circuit de mesure de température (41) en communication avec le capteur de température (40), le circuit de mesure de température (41) produisant une mesure de température à partir du signal de température.
